# FASCICULE DE BREVET EUROPEEN

(11) **EP 3 683 570 B1**
(45) Date de publication et mention de la délivrance du brevet: **20.11.2024**
(21) Numéro de dépôt: 20152008.7
(22) Date de dépôt: 15.01.2020
(51) Int. Cl.: G01N 21/53, G01N 21/47, F04B 49/10, F04D 15/02, G01N 15/02, G01N 29/02, F24H 1/00, F24D 3/00, F24D 19/10, G01N 33/18, F04D 15/00, F24D 19/00, G01N 15/00, G01N 15/06

(54) **DISPOSITIF DE MESURE DE LA QUALITE D'UN FLUIDE DANS UNE INSTALLATION HYDRAULIQUE**
VORRICHTUNG ZUM MESSEN DER QUALITÄT EINES FLUIDS IN EINER HYDRAULISCHEN ANLAGE
DEVICE FOR MEASURING THE QUALITY OF A FLUID IN A HYDRAULIC SYSTEM

(30) Priorité: 18.01.2019 FR 1900446
(43) Date de publication de la demande: 22.07.2020
(73) Titulaire: Wilo Intec, 18700 Aubigny sur Nere (FR)
(72) Inventeur: GONZALEZ, Philippe, 18110 Saint Martin d'Auxigny (FR); LE GALLO, Yann, 45000 Orléans (FR); MANA, Zakaria, 18000 BOURGES (FR)
(74) Mandataire: Hirsch & Partners

(56) Documents cités:
- DE-A1- 4 028 881
- JP-A- 2002 236 111
- US-A- 5 438 420
- US-A1- 2015 070 036
- US-A1- 2017 122 326
- US-A1- 2017 336 081

## Description

### Domaine de l'invention

La présente invention concerne un dispositif de mesure de la qualité d'un fluide d'une installation hydraulique, ainsi qu'une installation hydraulique comprenant un tel dispositif. Elle trouve notamment une application dans les domaines du chauffage, de la ventilation et/ou climatisation d'un bâtiment. L'installation hydraulique équipe en particulier des bâtiments industriels ou des habitats individuels.

### Etat de la technique

Dans le domaine des installations hydrauliques, il est connu de recourir à une pompe de circulation d'un fluide, également appelée circulateur, pour permettre la circulation d'un fluide dans l'installation hydraulique. De telles pompes sont ainsi présentes dans les installations d'eau chaude sanitaire ou dans les installations de chauffage domestique, qu'il s'agisse de chauffage au fioul, au gaz, au bois, par pellets ou par énergie solaire. De telles installations sont réalisées au domicile de l'utilisateur, directement dans la partie privative du domicile ou dans les parties communes d'une copropriété du domicile de l'utilisateur.

Une telle installation hydraulique comprend généralement un circulateur ou une pompe de circulation permettant de faire circuler le fluide, tel que de l'eau, dans la ou les voies de circulations de l'installation. Elle comprend par ailleurs un ou plusieurs éléments hydrauliques, comme par exemple un échangeur thermique du type chaudière et un radiateur. L'eau circule donc grâce à la pompe, passe dans la chaudière où elle y est chauffée avant d'arriver dans le radiateur permettant de diffuser la chaleur à l'extérieur. L'eau perd donc une quantité de sa chaleur diffusée dans le radiateur, puis retourne vers la chaudière pour y être à nouveau chauffée.

Si l'eau est de mauvaise qualité, le rendement calorifique baisse et l'efficacité de l'installation hydraulique est réduite. L'eau a en effet plus de mal à chauffer, ce qui sollicite la chaudière de façon anormale. L'eau peut aussi avoir plus de mal à circuler, ce qui sollicite la pompe de façon anormale. Et le résultat en termes de restitution d'une quantité de chaleur par le radiateur est insuffisant.

C'est le cas par exemple lorsque l'eau est polluée par des particules de matières quelconques.

Également, la présence de bulles d'air dans l'eau nuit à l'efficacité de l'installation hydraulique, et peut même être dommageable pour certains des éléments hydrauliques qui la composent. Dans le cas extrême d'une installation dont le circuit hydraulique n'est plus en eau mais en air, c'est-à-dire que l'eau a été complètement remplacée par de l'air, la mise en service de l'installation peut engendrer des dégâts sur certains des éléments hydrauliques qui la composent.

Certaines de ces installations hydrauliques comprennent un échangeur thermique type chaudière intelligente. Une telle chaudière est capable, après quelques secondes de mises en service de l'installation, de stopper son bruleur après avoir détecté l'absence d'eau dans le circuit. Cette détection correspond à une détection d'absence de charge, c'est-à-dire d'absence de résistance exercée sur la rotation de la pompe. Dans une telle installation, c'est donc la pompe qui fait remonter l'information, après plusieurs secondes de fonctionnement qui peuvent mener à la détérioration de la chaudière.

En outre, la détection d'absence de charge ne permet pas d'évaluer la présence de particules polluantes dans l'eau.

Par ailleurs, il est connu d'injecter certains produits chimiques dans une installation hydraulique, à des fins d'entretien ou de bon fonctionnement. Lorsque ces produits sont utilisés dans les bonnes proportions, ils permettent de protéger l'installation (absence de rouille et donc de particules susceptibles de circuler dans l'installation). Toutefois, si la qualité de l'eau est détériorée par la présence de particules ou autres produits chimiques, l'eau de l'installation voit son opacité modifiée. Cela peut survenir lorsque la date du dernier entretien est dépassée ou lorsqu'il survient un problème sur un des éléments de l'installation.

US 2017/122326 A1 concerne une pompe centrifuge et l'activation d'une pompe centrifuge à vitesse réglable et en fonction du nombre de particules détectées. DE 40 28 881 A1 concerne un procédé ainsi qu'un dispositif de protection d'un dispositif de transport contre la marche à sec, avec une conduite pour des fluides liquides ou gazeux, en particulier avec une pompe centrifuge transportant un liquide. US 2015/070036 A concerne un système de détection de fonctionnement à sec pour une pompe, ainsi qu'un ensemble de pompe. US 5 438 420

A concerne une méthode et un appareil de surveillance continue en ligne du niveau de contamination des fluides dans les systèmes hydrauliques et de lubrification. JP 2002 236111 concerne la méthode de détection des bulles d'une pompe. US 2017/336081 A1 concerne un système hydraulique de manière à réduire le nombre de dispositifs de commutation à actionnement électrique nécessaires dans le système.

Il est donc souhaitable de savoir dans quelle mesure l'eau est polluée par des particules de matières quelconques, ou la quantité de produits chimiques éventuels, qu'elle contient, pour définir des règles d'entretien et de gestion de l'installation hydraulique, et agir de manière préventive et non correctrice.

Également, il est souhaitable que la présence d'air dans l'eau, qui nuit à l'efficacité de l'installation hydraulique et peut même être dommageable pour certains des éléments hydrauliques qui composent cette installation, puisse être détectée le plus tôt possible.

### Objet de l'invention

Ainsi, l'invention a notamment pour objet de résoudre les problèmes précités, en permettant de détecter la présence de particules et/ou de produits chimiques et/ou d'air dans le fluide circulant dans l'installation hydraulique.

L'invention a donc pour objet, selon un premier aspect, un dispositif de mesure de la qualité d'un fluide selon la revendication 1.

Le dispositif comprend une pompe destinée à faire circuler le fluide dans l'installation hydraulique et comportant une unité de contrôle, et des moyens de mesure disposés sur une paroi extérieure de la pompe et reliés à l'unité de contrôle de la pompe et aptes à mesurer la proportion d'air, dans le fluide circulant dans l'installation hydraulique, l'unité de contrôle de la pompe étant configurée pour contrôler le fonctionnement de la pompe en fonction d'une information de proportion d'air mesurée par les moyens de mesure.

Suivant certains modes de réalisation, le dispositif comprend en outre une ou plusieurs des caractéristiques suivantes, prise(s) isolément ou suivant toutes les combinaisons techniquement possibles :
- les moyens de détermination sont des moyens de mesure aptes à mesurer la teneur en particules du fluide circulant dans l'installation hydraulique ;
- les moyens de détermination sont de type turbidimètre, aptes à émettre un signal lumineux en direction du fluide circulant dans l'installation et à mesurer la diffraction subie par le signal lumineux au contact dudit fluide ;
- les moyens de détermination sont aptes à émettre un signal sonore en direction du fluide circulant dans l'installation et à mesurer la réfraction subie par le signal sonore au contact dudit fluide.

L'invention a également pour objet, selon un deuxième aspect, une installation hydraulique comprenant au moins une voie de circulation d'un fluide, et comprenant un dispositif de mesure de la qualité d'un fluide selon la revendication 1, disposé en sorte de permettre de mesurer la qualité du fluide circulant dans ladite voie. Suivant certains modes de réalisation, l'installation comprend en outre une ou plusieurs des caractéristiques suivantes, prise(s) isolément ou suivant toutes les combinaisons techniquement possibles :
- l'installation hydraulique comprend au moins un élément hydraulique, tel qu'un échangeur thermique, et au moins un actionneur apte à piloter l'élément hydraulique, tel qu'un régulateur de chaudière, connecté à l'unité de contrôle de la pompe, en sorte de permettre l'échange d'information entre la pompe et l'actionneur ;
- l'installation hydraulique comprend au moins un élément hydraulique, tel qu'un échangeur thermique, et au moins un actionneur apte à piloter l'élément hydraulique, tel qu'un régulateur de chaudière, connecté aux moyens de détermination, en sorte de permettre l'échange d'information entre les moyens de détermination et l'actionneur ;
- l'actionneur est configuré pour contrôler le fonctionnement de l'élément hydraulique en fonction d'une information déterminée par les moyens de détermination.

Ainsi, le dispositif permet de mesurer la proportion d'air dans le circuit hydraulique de l'installation.

Cela permet notamment de maintenir un niveau d'efficacité élevé de l'installation hydraulique, en définissant des règles d'entretien et de gestion de cette installation, pour agir de manière préventive et non correctrice. Cela permet également de réduire les risques de détérioration.

Ainsi, l'invention peut permettre de détecter un changement d'opacité et donc de qualité d'eau, et permet de prévenir l'utilisateur de la nécessité d'une intervention préventive sur celle-ci.

### Description des dessins

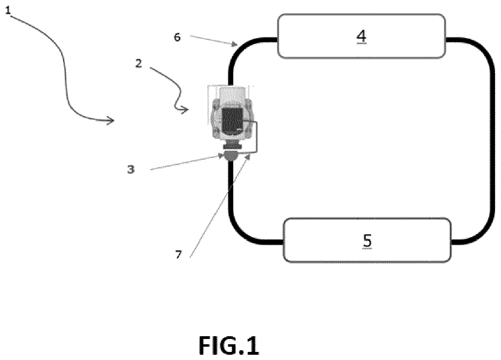

Les caractéristiques et avantages de l'invention apparaitront à la lecture de la description qui va suivre, donnée uniquement à titre d'exemple, et non limitative, en référence à la figure 1 annexée qui représente schématiquement un exemple d'installation hydraulique selon l'invention.

### Description détaillée

Sur la figure 1 est représenté un exemple d'installation hydraulique 1 comprenant un dispositif de mesure de la qualité du fluide circulant dans l'installation, différents éléments hydrauliques 4, 5, et une pompe 2 pour faire circuler le fluide dans l'installation 1 au moyen de canalisations 6.

Précisément, il s'agit d'une installation hydraulique de chauffage comprenant un échangeur thermique 4 (qui permet l'apport de l'énergie dans l'installation) tel qu'une chaudière 4 et un radiateur 5 (qui permet la dissipation de la chaleur vers l'extérieur), dans laquelle circule de l'eau.

La pompe 2 comprend une unité électronique de contrôle, configurée pour gérer l'ensemble du fonctionnement de cette pompe 2.

Des moyens de détermination 3 permettent de mesurer la proportion d'air, et éventuellement, en plus, de mesurer la teneur en particules données, dans le fluide circulant dans l'installation hydraulique 1.

Il peut s'agir par exemple d'un dispositif de type turbidimètre 3 qui utilise la diffraction de la lumière pour déterminer la présence de particules ou de produits chimiques donnés ou de bulles d'air dans l'eau. Le turbidimètre 3 émet un signal lumineux en direction du fluide circulant dans l'installation 1 et mesure la diffraction subie par le signal lumineux au contact du fluide contenu dans cette canalisation (qui peut être de l'eau ou de l'air).

Dans l'exemple représenté sur la figure 1, hors de l'étendue des revendications, le turbidimètre 3 est placé sur l'une des canalisations de l'installation 1, à proximité de la pompe 2. Selon l'invention, il est placé directement sur une paroi externe de la pompe 2.

Le turbidimètre 3 est relié à l'unité de contrôle de la pompe 2 par un câble 7. Alternativement, la connexion entre l'unité de contrôle de la pompe 2 et le turbidimètre 3 pourrait être une connexion sans fil.

L'unité de contrôle de la pompe 2 est configurée pour contrôler le fonctionnement de la pompe 2 en fonction d'une information de proportion d'air mesurée par les moyens de mesure.

Le turbidimètre 3 peut mesurer la teneur précise de particules données dans l'eau, et la proportion précise d'air contenu dans l'eau.

Un au moins des éléments hydraulique 4, 5 est piloté par un actionneur. Dans l'exemple de la figure 1, la chaudière 4 est pilotée par un régulateur de chaudière. Ce régulateur est connecté à l'unité de contrôle de la pompe 2. Il peut ainsi recevoir des informations telles que les informations déterminées par le turbidimètre 3, via l'unité de contrôle de la pompe 2. Le régulateur peut aussi être connecté directement, par voie filaire ou non filaire, au turbidimètre, et recevoir ainsi directement les informations déterminées par ce turbidimètre 3.

Le régulateur de chaudière est configuré pour contrôler le fonctionnement de la chaudière 4 en fonction des informations déterminées par le turbidimètre 3.

Par exemple, lorsque le turbidimètre détermine une proportion d'air trop importante dans l'eau, le régulateur de la chaudière 4 en est informé immédiatement et peut stopper le brûleur de la chaudière 4 pour éviter tout endommagement. De même, en cas d'absence totale d'eau dans le circuit hydraulique, le turbidimètre 3 le détecte et la chaudière 4 est mise en sécurité avant même que la pompe 2 ne fasse remonter une information d'absence ou de perte de charge, ce qui permet d'éviter une détérioration de la chaudière 4.

La présente description est donnée à titre d'exemple et n'est pas limitative de l'invention qui n'est définie que par les revendications annexées.

En particulier, d'autres technologies qu'un turbidimètre peuvent être utilisées, tel que des moyens d'émission d'un signal sonore et de mesure de la réfraction de ce signal au contact de l'eau.

## Revendications

1. Dispositif de mesure de la qualité d'un fluide circulant dans au moins une voie d'une installation hydraulique (1), le dispositif comprenant une pompe (2) destinée à faire circuler le fluide dans l'installation hydraulique (1) et comportant une unité de contrôle, et des moyens de mesure (3) disposés sur une paroi extérieure de la pompe (2) et reliés à l'unité de contrôle de la pompe (2) et aptes à mesurer la proportion d'air, dans le fluide circulant dans l'installation hydraulique (1), l'unité de contrôle de la pompe (2) étant configurée pour contrôler le fonctionnement de la pompe (2) en fonction d'une information de proportion d'air mesurée par les moyens de mesure (3).

2. Dispositif selon la revendication 1, **caractérisé en ce que** les moyens de mesure (3) sont aptes à mesurer la teneur en particules données dans le fluide circulant dans l'installation hydraulique (1), l'unité de contrôle de la pompe (2) étant configurée pour contrôler le fonctionnement de la pompe (2) en fonction d'une information de teneur en particules données mesurée par les moyens de mesure (3).

3. Dispositif selon l'une quelconque des revendications 1 et 2, **caractérisé en ce que** les moyens de mesure (3) sont de type turbidimètre, aptes à émettre un signal lumineux en direction du fluide circulant dans l'installation (1) et à mesurer la diffraction subie par le signal lumineux au contact dudit fluide.

4. Dispositif selon l'une quelconque des revendications 1 à 3, **caractérisé en ce que** les moyens de mesure (3) sont aptes à émettre un signal sonore en direction du fluide circulant dans l'installation (1) et à mesurer la réfraction subie par le signal sonore au contact dudit fluide.

5. Installation hydraulique (1) comprenant au moins une voie de circulation d'un fluide, **caractérisée en ce qu'**elle comprend un dispositif de mesure de la qualité d'un fluide selon l'une quelconque des revendications précédentes, disposé en sorte de permettre de mesurer la qualité du fluide circulant dans ladite voie.

6. Installation hydraulique (1) selon la revendication 5, **caractérisée en ce qu'**elle comprend au moins un élément hydraulique (4), tel qu'un échangeur thermique (4), et au moins un actionneur apte à piloter l'élément hydraulique (4), tel qu'un régulateur de chaudière, connecté à l'unité de contrôle de la pompe (2), en sorte de permettre l'échange d'information entre la pompe (2) et l'actionneur.

7. Installation hydraulique (1) selon l'une quelconque des revendications 5 ou 6, **caractérisée en ce qu'**elle comprend au moins un élément hydraulique (4), tel qu'un échangeur thermique (4), et au moins un actionneur apte à piloter l'élément hydraulique (4), tel qu'un régulateur de chaudière, connecté aux moyens de détermination (3), en sorte de permettre l'échange d'information entre les moyens de mesure (3) et l'actionneur.

8. Installation hydraulique (1) selon la revendication 7, **caractérisée en ce que** l'actionneur est configuré pour contrôler le fonctionnement de l'élément hydraulique (4) en fonction de d'une information mesurée par les moyens de mesure (3).

## Patentansprüche

1. Vorrichtung zur Messung der Qualität eines Fluids, das in mindestens einem Weg einer hydraulischen Anlage (1) zirkuliert, wobei die Vorrichtung eine Pumpe (2), die dazu bestimmt ist, das Zirkulieren des Fluids in der hydraulischen Anlage (1) zu bewirken, und eine Steuereinheit aufweist, und Messmittel (3), die an einer Außenwand der Pumpe (2) angeordnet sind und mit der Steuereinheit der Pumpe (2) verbunden sind und geeignet sind, den Luftanteil in dem in der hydraulischen Anlage (1) zirkulierenden Fluid zu messen, umfasst, wobei die Steuereinheit der Pumpe (2) dafür ausgelegt ist, den Betrieb der Pumpe (2) in Abhängigkeit von einer Information über den von den Messmitteln (3) gemessenen Luftanteil zu steuern.

2. Vorrichtung nach Anspruch 1, **dadurch gekennzeichnet, dass** die Messmittel (3) geeignet sind, den Gehalt an gegebenen Partikeln in dem in der hydraulischen Anlage (1) zirkulierenden Fluid zu messen, wobei die Steuereinheit der Pumpe (2) dafür ausgelegt ist, den Betrieb der Pumpe (2) in Abhängigkeit von einer Information über den von den Messmitteln (3) gemessenen Gehalt an gegebenen Partikeln zu steuern.

3. Vorrichtung nach einem der Ansprüche 1 und 2, **dadurch gekennzeichnet, dass** die Messmittel (3) vom Typ eines Trübungsmessers sind und geeignet sind, ein Lichtsignal in Richtung des in der Anlage (1) zirkulierenden Fluids auszusenden und die Beugung zu messen, die das Lichtsignal im Kontakt mit dem Fluid erfährt.

4. Vorrichtung nach einem der Ansprüche 1 bis 3, **dadurch gekennzeichnet, dass** die Messmittel (3) geeignet sind, ein Schallsignal in Richtung des in der Anlage (1) zirkulierenden Fluids auszusenden und die Brechung zu messen, die das Schallsignal im Kontakt mit dem Fluid erfährt.

5. Hydraulische Anlage (1), welche mindestens einen Zirkulationsweg eines Fluids umfasst, **dadurch gekennzeichnet, dass** sie eine Vorrichtung zur Messung der Qualität eines Fluids nach einem der vorhergehenden Ansprüche umfasst, die so angeordnet ist, dass sie ermöglicht, die Qualität des in dem Weg zirkulierenden Fluids zu messen.

6. Hydraulische Anlage (1) nach Anspruch 5, **dadurch gekennzeichnet, dass** sie mindestens ein hydraulisches Element (4) umfasst, wie etwa einen Wärmetauscher (4), und mindestens einen Aktuator, der geeignet ist, das hydraulische Element (4) zu steuern, wie etwa einen Kesselregler, der mit der Steuereinheit der Pumpe (2) verbunden ist, derart, dass der Informationsaustausch zwischen der Pumpe (2) und dem Aktuator ermöglicht wird.

7. Hydraulische Anlage (1) nach einem der Ansprüche 5 oder 6, **dadurch gekennzeichnet, dass** sie mindestens ein hydraulisches Element (4) umfasst, wie etwa einen Wärmetauscher (4), und mindestens einen Aktuator, der geeignet ist, das hydraulische Element (4) zu steuern, wie etwa einen Kesselregler, der mit den Bestimmungsmitteln (3) verbunden ist, derart, dass der Informationsaustausch zwischen den Messmitteln (3) und dem Aktuator ermöglicht wird.

8. Hydraulische Anlage (1) nach Anspruch 7, **dadurch gekennzeichnet, dass** der Aktuator dafür ausgelegt ist, den Betrieb des hydraulischen Elements (4) in Abhängigkeit von einer von den Messmitteln (3) gemessenen Information zu steuern.

## Claims

1. Device for measuring the quality of a fluid circulating in at least one channel of a hydraulic installation (1), the device comprising a pump (2) intended to circulate the fluid in the hydraulic installation (1) and comprising a control unit, and measuring means (3) arranged on an outer wall of the pump (2) and connected to the control unit of the pump (2) and capable of measuring the proportion of air in the fluid circulating in the hydraulic installation (1), the control unit of the pump (2) being configured to control the operation of the pump (2) as a function of information on the proportion of air measured by the measuring means (3).

2. Device according to claim 1, **characterized in that** the measuring means (3) are capable of measuring the content of given particles in the fluid circulating in the hydraulic installation (1), the pump (2) control unit being configured to control the operation of the pump (2) according to information on the content of given particles measured by the measuring means (3).

3. Device according to any one of claims 1 and 2, **characterized in that** the measuring means (3) are of the turbidimeter type, capable of emitting a light signal towards the fluid circulating in the installation (1) and of measuring the diffraction undergone by the light signal upon contact with said fluid.

4. Device according to any one of claims 1 to 3, **characterized in that** the measuring means (3) are capable of emitting an audible signal towards the fluid circulating in the installation (1) and of measuring the refraction undergone by the audible signal upon contact with said fluid.

5. Hydraulic installation (1) comprising at least one fluid circulation path, **characterized in that** it comprises a device for measuring the quality of a fluid according to any one of the preceding claims, arranged so as to allow the quality of the fluid circulating in said path to be measured.

6. Hydraulic installation (1) according to claim 5, **characterized in that** it comprises at least one hydraulic element (4), such as a heat exchanger (4), and at least one actuator capable of controlling the hydraulic element (4), such as a boiler regulator, connected to the control unit of the pump (2), so as to allow the exchange of information between the pump (2) and the actuator.

7. Hydraulic installation (1) according to any one of claims 5 or 6, **characterized in that** it comprises at least one hydraulic element (4), such as a heat exchanger (4), and at least one actuator capable of controlling the hydraulic element (4), such as a boiler regulator, connected to the determination means (3), so as to allow the exchange of information between the measuring means (3) and the actuator.

8. Hydraulic installation (1) according to claim 7, **characterized in that** the actuator is configured to control the operation of the hydraulic element (4) as a function of information measured by the measuring means (3).
